# EUROPEAN PATENT SPECIFICATION

(11) **EP 3 258 976 B1**
(45) Date of publication and mention of the grant of the patent: **24.07.2024**
(21) Application number: 16718001.7
(22) Date of filing: 16.02.2016
(51) Int. Cl.: A61L 27/16, A61L 27/46, A61L 27/54, A61L 27/56, B33Y 80/00, B33Y 70/10

(54) **MATERIAL FOR THE MOLDING OF DEVICES TO BE IMPLANTED INTO THE HUMAN BODY OR OF ARTICULAR SPACERS**
MATERIAL ZUM FORMEN VON VORRICHTUNGEN ZUR IMPLANTATION IN DEN MENSCHLICHEN KÖRPER ODER VON GELENKABSTANDSHALTERN
MATÉRIAU POUR LE MOULAGE DE DISPOSITIFS DESTINÉS À ÊTRE IMPLANTÉS DANS LE CORPS HUMAIN OU D'ESPACEURS ARTICULAIRES

(30) Priority: 16.02.2015 IT VR20150022
(43) Date of publication of application: 27.12.2017
(73) Proprietor: Tecres S.P.A., 37066 Sommacampagna (VR) (IT)
(72) Inventor: FACCIOLI, Giovanni, 37066 Sommacampagna (Verona) (IT); SOFFIATTI, Renzo, 37066 Sommacampagna (Verona) (IT)
(74) Representative: Feltrinelli, Secondo Andrea
(86) International application number: PCT/IB2016/050819
(87) International publication number: WO 2016/132289

(56) References cited:
- EP-A1- 0 472 237
- WO-A1-2010/096053
- WO-A1-2012/007535
- WO-A1-2014/075185
- WO-A2-2013/184010
- DE-A1- 102007 052 519
- US-B1- 6 641 831

## Description

### TECHNICAL FIELD OF THE INVENTION

The present invention concerns a biocompatible and implantable material to be used to mould devices able to be implanted in the human body such as spacer devices, prostheses, etc.

### STATE OF THE ART

The technology of additive production or three-dimensional printing of objects is currently becoming increasingly popular.

In particular, three-dimensional or 3D printing, which represents a natural evolution of 2D printing, allows an object to be reproduced from a three-dimensional model through the superimposition of successive layers of material, until the desired shape is obtained. Such a three-dimensional model is obtained through suitable software.

3D printers are generally faster, more reliable and simpler to use with respect to other technologies for additive production and, moreover, offer the possibility of moulding and assembling parts made up of different materials with different physical and mechanical properties in a single construction process.

The operation of a three-dimensional printer is based on the use of a 3D file developed by suitable software.

The 3D model of the object of interest is prepared - through the aforementioned software - in a series of portions or layers in cross section thereof.

Each portion or layer is then printed one onto the other, in order to recreate the entire three-dimensional object.

There are different 3D printing technologies; their main differences concern the way in which the various layers are printed.

Some methods, in order to produce the various layers, use materials that melt or soften. Some examples of such technology are "Selective Laser Sintering" (SLS) or "Direct Metal Laser Sintering" (DMLS) or "Fused Deposition Modeling" (FDM).

Other methods provide for the deposition layer-by-layer of liquid materials, which are made to set with different technologies. An example of such a technology is known as "Digital Light Processing" (DLP).

Furthermore, in the case of lamination systems there are thin layers that are cut according to the desired shape and then joined together through known techniques.

A first 3D printing method consists of a printing system by extrusion of material. The printer creates the model one layer at a time, spreading a layer of powder (plaster or resins) and distributing a binder thereupon, for example printing like with an inkjet.

The process is repeated layer by layer until the desired shape of the object is obtained.

In this way, it is possible to make projections in the finished product.

FDM technology, used in conventional quick prototyping, provides for a nozzle adapted for depositing a molten polymer layer-by-layer on a support structure.

Furthermore, some 3D printers for additive synthesis use a thread of thermoplastic polymer as construction material.

In the field of orthopaedics, three-dimensional printing technology has led to bone or parts thereof being made. For example, it has been possible to implant a cranium printed in 3D to a patient.

The skullcap was made with a special resin through the use of a 3D printer. Other recently-developed prostheses were obtained through three-dimensional printing of a titanium-based material.

The materials usually used for 3D printing are: plastic materials, for example thermoplastic polymers (for example for SLS and FDM), metals, sand, glass (for example for SLS), photopolymers (for example for stereolithography), laminated sheets (often of the paper type) and relative glues, titanium alloys (for example for "Electron beam melting" or EBM), resins, clays, ceramic, etc.

In particular, for jet or thread 3D printing, the material used must be melted and extrudable through a nozzle suitable for the purpose.

Therefore, one of the problems to be solved in order to be able to three-dimensionally print some objects, particularly for medical or orthopaedic use, is precisely that of making materials having the appropriate characteristics for the final purpose for which the object must be made and at the same time characteristics suitable for the selected three-dimensional printing method.

Basically, surgical practice provides for implanting resins or plastic materials in the body for a short time (for example for catheters) or permanently (for example for polymethyl methacrylate or PMMA, constituent of bone cement, for ultra high molecular weight polyethylene or UHMWPE or in short PE, as friction surface in hip, knee, shoulder prostheses etc., or for polyetheretherketone or PEEK, as cranial prostheses, spinal cages, etc.).

Except for the PMMA of bone cement, all of the other plastic materials undergo mechanical processing in order to obtain a prosthesis or a device to be implanted in the human body.

This occurs both due to objective difficulty in moulding those specific plastic materials, and particularly for the extremely heterogeneous demand of prostheses or devices to be implanted in the human body of greatly different shape and size, requirements which only mechanical processing can quickly satisfy.

However, these plastic materials (for example PE and PEEK) do not and cannot contain pharmaceutical or medical substances, like for example one or more antibiotics, nor radio-opacifying agents, like for example barium, nor possible further medical additives used to treat the patient or for surgical treatment.

Such substances, however, would be very useful and appreciated inside such plastics in order to carry out a healing function (for example local antibiotic) and be easily detected radiographically, as done on the other hand by antibiotic-loaded bone cement provided with radio-opacifying agents.

Therefore, there is a need for a biocompatible and implantable material, added to with pharmaceutical or medical substances and/or radio-opacifying agents and/or other useful additives, to be used for moulding, also three-dimensional, of devices to be implanted in the human body or spacer devices.

The application WO2012/007535 discloses a part for endosseous implantation molded through injection moulding of a material comprising a thermoplastic binder (preferably PEEK) that incorporate fibers; TCP and zeolite can be incorporated into the binder, the latter substance being able to confer radiopacity at the implant.

The application EP0472237 discloses a material comprising UHMWPE and inorganic filler such as calcium phosphate or hydroxyapatite.

The application DE102007052519 discloses a medical implant, comprising a polymeric material (such as polyethylene or polypropylene) and an antimicrobial composition (including silicon dioxide and metal-containing nanoparticles).

The application WO2010/096053 discloses a medical Implant incorporating a medical substance, such as silver or penicillin.

The application WO2013/184010 discloses a middle ear prosthesis including silver powder.

The patent US6641831 discloses a non-degradable medical product comprising at least two substances: substance A and substance B, wherein substance A is more lipophilic than substance B and has a given solubility in water, lower than that of substance B. At least one, among substance A and substance B, is a pharmaceutically active agent, i.e. an antimicrobial substance.

WO 2014/075185 describes implantable medical devices provided with antimicrobial properties and formed by additive manufacturing technologies.

### AIMS OF THE INVENTION

The task of the present invention is to improve the state of the art.

In such a technical task, an aim of the present invention is to provide a biocompatible and implantable material, adapted for being used in three-dimensional printing, and which can be added to with pharmaceutical or medical substances and with further additives. An aim of the present invention is to provide a biocompatible and implantable material that is extrudable.

The present invention is defined by the independent claims, while the dependent claims concern the preferred embodiments. Any "embodiment" or "example" which is disclosed in the description but is not covered by the claims should be considered as presented for illustrative purpose only.

In accordance with an aspect of the present invention, a biocompatible and implantable material in the form of a thread used to feed a three-dimensional printer of the thread or inkjet type is provided according to claim 1.

In accordance with another aspect of the present invention, a device to be implanted in the human body or a spacer device for the treatment of a bone or joint location made with a biocompatible and implantable material is provided, wherein said device is obtained by molding the thread of claim 1 by means of a tridimensional printer, as defined in claim 6.

An advantage of such a device to be implanted in the human body or a spacer device for the treatment of a bone or joint seat consists of being able to be added to with pharmaceutical or medical substances and with additives and be made through three-dimensional moulding.

A further advantage of the device to be implanted in the human body or a spacer device for the treatment of a bone or joint location consists of being able to be personalised or made in series in a quick and simple manner, substantially without the need for further surface finishing processing.

In accordance with another aspect of the present invention a method for obtaining a device that can be implanted in the human body according to claim 6 is provided in claim 9.

An advantage of such a method is that it is quick and simple, substantially without the need for further surface finishing processing steps.

The dependent claims refer to preferred and advantageous embodiments of the invention.

### EMBODIMENTS OF THE INVENTION

The present invention refers to a material biocompatible and implantable in the human body, for the obtainment of a device that can be implanted in the human body or a spacer device for treating a bone or a joint seat.

Such a material comprises low density polyethylene or high density polyethylene or ultra high molecular weight polyethylene (UHMWPE) or a mixture thereof.

The plastic material may further comprise an acrylic resin comprising an acrylic copolymer made up of MMA, styrene and ethyl-acrylate or polymethyl methacrylate or mixtures comprising acrylic polymers and/or co-polymers.

One of the main characteristics of such a material is that it is mouldable through three-dimensional printing technology.

In this way it is possible to mould such a material according to a simple and quick procedure, capable of providing both medical devices or spacer devices produced in series, and personalised devices.

In the latter case, indeed, it is possible to obtain a three-dimensional model of the device to be obtained, by selecting the size and shape or configuration most suitable for the surgical or anatomical requirements of the patient, and produce the relative device adapted to the specific patient.

In the aforementioned cases, however, thanks to the mouldability of the material according to the present invention, it is possible to obtain finished devices, substantially without the need to carry out further surface finishing or processing steps thereof.

The material according to the present invention also comprises gentamicin sulphate as a pharmaceutical or medical substance and a radio-opacifying agent and a further additive comprising a soluble and/or resorbable ceramic material, in form of powder or granules, that is tricalcium phosphate or calcium sulphate or hydroxyapatite.

Such an option is particularly relevant when the device to be obtained with said material is a spacer device for treating an infection present in a bone or joint location.

The function of spacer devices, indeed, is to maintain the joint space left by an infected prosthesis, which for this reason is removed, and at the same time to treat the infection of the bone location, internally comprising a pharmaceutical or medical substance, as defined in the independent claims, to be eluted in the area to be treated.

The material according to the present invention also comprises a radio-opacifying agent, like for example metallic powders, for example tungsten, tantalum, silver or salts such as barium sulphate, zirconium oxide, bismuth oxide, etcetera.

Such agents, as known, are visible to X-rays and thus make it possible to monitor the position of the device to be implanted in the human body or the spacer device, as well as the material that contains them according to the present invention.

The material according to the present invention, moreover, comprises further medical additives in the form of a soluble and/or reabsorbable ceramic material, in the form of powder or granules, such as tricalcium phosphate or calcium sulphate or hydroxyapatite.

One of the problems to be solved, for the material according to the present invention, is that the substances contained therein can be degraded or undergo modifications due to the temperatures and/or pressures that the material according to the present invention encounters, when the material is heated or when it is moulded through three-dimensional printing.

The material according to the present invention is in the form of a thread, for example having a diameter that can vary between 1 and 10 millimetres or between 1 and 5 millimetres, adapted for being used to feed a three-dimensional printer of the thread or inkjet type.

One of the methods for allowing the material according to the present invention to be reduced to a thread adapted for being printed three-dimensionally is as follows.

The base material is provided, for example in the form of a pellet (e.g. press pellet) or of granules; the material is inserted in suitable machinery, for example an extruder, and an additive, i.e. the gentamicin sulphate as pharmaceutical or medical substance and a radio-opacifying agent and a further additive comprising a soluble and/or resorbable ceramic material, in form of powder or granules, that is tricalcium phosphate or calcium sulphate or hydroxyapatite, is added.

Then the whole thing is heated until a certain temperature is reached, for example the melting temperature thereof, or to a temperature suitable for melting or softening the material in question (together with the mixture of additives that are added), to such a point as to be able to be extruded in threads or mouldable.

Such a melting temperature varies as the polymers comprised according to the present invention varies. Most of such polymers have melting values comprised between 60° and 300°. Such a melting temperature can, for example, be around 250°C.

According to the claimed invention, there is then a step of extruding the heated or molten material through suitable machinery, for example the same extruder, in the form of one or more threads having the diameter indicated above.

Such at least one thread can be wound in a coil so that, being extruded, it cools down and becomes consolidated and thus is suitable to then be handled and stored.

Such at least one thread is then used to feed a nozzle of a 3D printer.

Such a thread is moulded through a three-dimensional printer in order to obtain a device that can be implanted in the human body or a spacer device for treating a bone or a joint location.

As stated, the temperature at which the material is melted must be below the degradation or damaging temperature of the pharmaceutical or medical substance, or of the radio-opacifying agent or of the further additive, so that they maintain their original characteristics also, following melting of the material, in the thread or resulting material.

For example, an antibiotic that has a melting temperature of 180°C, so as not to degrade during moulding, must not exceed such a temperature.

Through the aforementioned material it is possible to obtain devices that can be implanted in the human body for which it may be useful to have a medicated version, an X-ray visible version, a coloured version, etcetera.

According to the present invention, the device implantable in the human body or spacer device for treating an infection in a bone or joint location is porous. The porosity of the device makes it capable of absorbing, for example by capillary action, further pharmaceutical or medical substances after it has been formed.

These devices are made with gentamicin sulphate already being comprised within the device but, being porous, once formed they are capable of absorbing another substance, the same or different with respect to the one already contained in them.

In this way, it has been seen how the material according to the present invention, being able to be moulded and made in the form of a thread, allows devices able to be implanted in the human body or spacer devices to be obtained in a quick, simple and possibly personalisable manner both in terms of the shape and the size, and also the pharmaceutical or medical substances and the further additives or agents contained therein, according to the surgical or anatomical requirements of the patient.

The present invention also refers to a device that can be implanted in the human body or to a spacer device for treating a bone or a joint location, comprising a material biocompatible and implantable in the human body according to the present invention.

Such devices comprise gentamicin sulphate as a pharmaceutical or medical substance and a radio-opacifying agent and a further additive, as described earlier for the material according to the present invention.

Such devices are made by moulding through a three-dimensional printer.

Another embodiment that can be obtained with the material according to the present invention is a cranial prosthesis. In this way, the prosthesis could be made directly from the CAT data, formed according to the configuration and the dimensions necessary for the anatomical and implanting requirements, and then implanted in the bone location of interest.

Such a cranial prosthesis then contains a pharmaceutical or medical substance and a radio-opacifying agent and a further additive.

## Claims

1. Material biocompatible and implantable in the human body in the form of a thread used to feed a three-dimensional printer of the thread or inkjet type, for the obtainment of a device that can be implanted in the human body or a spacer device for treating an infection in a bone or a joint location, comprising a plastic material comprising low density polyethylene or high density polyethylene or ultra high molecular weight polyethylene (UHMWPE) or a mixture of the above, **characterized in that** it comprises an additive, wherein said additive is a pharmaceutical or medical substance comprising gentamicin sulphate, and a radio-opacifying agent, **in that** said biocompatible material is moldeable and it is extruded into said thread **and in that** said biocompatible material comprises a further additive comprising a soluble and/or resorbable ceramic material, in form of powder or granules, that is tricalcium phosphate or calcium sulphate or hydroxyapatite.

2. Material according to claim 1, wherein said thread has a diameter comprised between 1 and 10 mm or between 1 and 5 mm.

3. Material according to claim 1, wherein said biocompatible material is moldeable by means of a tridimensional printer.

4. Material according to claim 1, wherein said radio-opacifying agent is tungsten, tantalum or silver metallic powders or their salts or barium sulphate, zirconium oxide, bismuth oxide.

5. Material according to claim 1, wherein said plastic material further comprises an acrylic resin comprising an acrylic copolymer comprising MMA, styrene and ethyl acrylate or polymethylmethacrylate or mixtures comprising acrylic polymers and/or copolymers.

6. Device implantable in the human body or spacer device for treating an infection in a bone or a joint location, comprising a biocompatible material, wherein said biocompatible material comprises a plastic material comprising low density polyethylene or high density polyethylene or ultra high molecular weight polyethylene (UHMWPE) or a mixture of the above, **characterized in that** said biocompatible material comprises an additive, wherein said additive is a pharmaceutical or medical substance comprising gentamicin sulphate and a radio-opacifying agent, wherein said device is obtained by extruding said biocompatible material into a thread and then by molding said thread by means of a tridimensional printer, **in that** said device comprises a further additive comprising a soluble and/or reabsorbable ceramic material, in form of powder or granules, that is tricalcium phosphate or calcium sulphate or hydroxyapatite, **and in that** said device is porous and, once formed, is capable of absorbing another pharmaceutical or medical substance, the latter being gentamicin sulphate or different from gentamicin sulphate contained in said device.

7. Device according to claim 6, wherein said radio-opacifying agent is tungsten, tantalum or silver metallic powders or salts, barium sulphate, zirconium oxide, bismuth oxide.

8. Device according to any one of the claims 6 or 7, wherein said plastic material further comprises an acrylic resin comprising a copolymer composed of MMA, styrene and ethyl acrylate or polymethylmethacrylate or mixtures comprising acrylic polymers and/or copolymers.

9. Method for the obtainment of a device that can be implanted in the human body according to any one of the preceding claims 6 to 8, comprising the following steps:
providing a plastic material in form of granules comprising low density polyethylene or high density polyethylene or ultra high molecular weight polyethylene (UHMWPE) or a mixture of the above, wherein said material can be molded,
providing an additive, wherein said additive is a pharmaceutical or medical substance comprising gentamicin sulphate and a radio-opacifying agent,
providing a further additive comprising a soluble and/or resorbable ceramic material, in form of powder or granules, that is tricalcium phosphate or calcium sulphate or hydroxyapatite,
mixing or adding said plastic material and said additive obtaining a mixed or added base material,
heating said mixed or added base material to a predetermined temperature and extruding the melted material in shape of one or more threads used to feed a three-dimensional printer of the thread or inkjet type,
comprising a step of molding said thread by means of a tridimensional printer, in order to obtain a device that can be implanted in the human body or a spacer device in order for treating an infection in a bone or a joint location, wherein said device is porous and, once formed, is capable of absorbing another pharmaceutical or medical substance, the latter being gentamicin sulphate or different from gentamicin sulphate contained in said device.

10. Method according to claim 9, wherein said mixing or adding step and said heating step of said material occur simultaneously in a dedicated machinery, that is an extruder.

## Patentansprüche

1. Biokompatibles und in den menschlichen Körper implantierbares Material in Form eines Strangs, das zur Beschickung eines dreidimensionalen Druckers vom Strang- oder Tintenstrahl-Typ zur Erhaltung einer Vorrichtung, die in den menschlichen Körper implantiert werden kann, oder einer Abstandsvorrichtung zur Behandlung einer Infektion in einem Knochen oder einer Gelenkstelle verwendet wird, umfassend ein Kunststoffmaterial umfassend niedrigdichtes Polyethylen oder hochdichtes Polyethylen oder ultrahochmolekulargewichtiges Polyethylen (UHMWPE) oder eine Mischung der oben genannten, **dadurch gekennzeichnet, dass** es ein Additiv umfasst, worin das besagte Additiv eine pharmazeutische oder medizinische Substanz umfassend Gentamicinsulfat und ein strahlenundurchlässiges Mittel ist, dadurch, dass das besagte biokompatible Material formbar ist und zu dem besagten Strang extrudiert wird und dadurch, dass das besagte biokompatible Material ein weiteres Additiv umfassend ein lösliches und/oder resorbierbares keramisches Material in Form von Pulver oder Granulat, das Tricalciumphosphat oder Calciumsulfat oder Hydroxylapatit ist, umfasst.

2. Material nach Anspruch 1, worin der besagten Strang einen Durchmesser zwischen 1 und 10 mm oder zwischen 1 und 5 mm aufweist.

3. Material nach Anspruch 1, worin das besagte biokompatible Material mittels eines dreidimensionalen Druckers formbar ist.

4. Material nach Anspruch 1, worin das besagte strahlenundurchlässige Mittel Wolfram-, Tantal- oder Silbermetallpulver oder deren Salze oder Bariumsulfat, Zirkoniumoxid, Bismutoxid ist.

5. Material nach Anspruch 1, worin das besagte Kunststoffmaterial ferner ein Acrylharz umfassend ein Acrylcopolymer umfassend MMA, Styrol und Ethylacrylat oder Polymethylmethacrylat oder Mischungen umfassend Acrylpolymere und/oder -copolymere umfasst.

6. In den menschlichen Körper implantierbare Vorrichtung oder Abstandsvorrichtung zur Behandlung einer Infektion in einem Knochen oder einer Gelenkstelle, umfassend ein biokompatibles Material, worin das besagte biokompatible Material ein Kunststoffmaterial umfassend niedrigdichtes Polyethylen oder hochdichtes Polyethylen oder ultrahochmolekulargewichtiges Polyethylen (UHMWPE) oder eine Mischung der oben genannten umfasst, **dadurch gekennzeichnet, dass** das besagte biokompatible Material ein Additiv umfasst, worin das besagte Additiv eine pharmazeutische oder medizinische Substanz umfassend Gentamicinsulfat und ein strahlenundurchlässiges Mittel umfasst, worin die besagte Vorrichtung durch Extrudieren des besagten biokompatiblen Materials zu einem Strang und dann durch Formen des besagten Strangs mittels eines dreidimensionalen Druckers erhalten wird, dadurch, dass die besagte Vorrichtung ein weiteres Additiv umfassend ein lösliches und/oder resorbierbares keramisches Material in Form von Pulver oder Granulat, das Tricalciumphosphat oder Calciumsulfat oder Hydroxylapatit ist, umfasst und dadurch, dass die besagte Vorrichtung porös ist und, sobald sie geformt ist, in der Lage ist, eine andere pharmazeutische oder medizinische Substanz zu absorbieren, wobei letztere Gentamicinsulfat ist oder sich von dem in der besagten Vorrichtung enthaltenen Gentamicinsulfat unterscheidet.

7. Vorrichtung nach Anspruch 6, worin das besagte strahlenundurchlässige Mittel Wolfram-, Tantal- oder Silbermetallpulver oder -salze, Bariumsulfat, Zirkoniumoxid, Bismutoxid ist.

8. Vorrichtung nach irgendeinem der Ansprüche 6 oder 7, worin das besagte Kunststoffmaterial ferner ein Acrylharz umfassend ein Copolymer bestehend aus MMA, Styrol und Ethylacrylat oder Polymethylmethacrylat oder Mischungen umfassend Acrylpolymere und/oder -copolymere umfasst.

9. Verfahren zur Erhaltung einer Vorrichtung, die in menschlichen Körper implantiert werden kann, nach irgendeinem der vorangegangenen Ansprüche 6 bis 8, umfassend die folgenden Schritte:
des Bereitstellens eines Kunststoffmaterials in Form von Granulat umfassend niedrigdichtes Polyethylen oder hochdichtes Polyethylen oder ultrahochmolekulargewichtiges Polyethylen (UHMWPE) oder eine Mischung der oben genannten, worin das besagte Material geformt werden kann,
des Bereitstellens eines Additivs, worin das besagte Additiv eine pharmazeutische oder medizinische Substanz umfassend Gentamicinsulfat und ein strahlenundurchlässiges Mittel ist,
des Bereitstellens eines weiteren Additivs umfassend ein lösliches und/oder resorbierbares keramisches Material in Form von Pulver oder Granulat, das Tricalciumphosphat oder Calciumsulfat oder Hydroxylapatit ist,
des Mischens oder Zugebens des besagten Kunststoffmaterials und des besagten Additivs, wodurch ein gemischtes oder zugegebenes Basismaterial erhalten wird,
des Erhitzens des gemischten oder zugegebenen Basismaterials auf eine vorbestimmte Temperatur und des Extrudierens des geschmolzenen Materials in Form eines oder mehrerer Stränge, die zur Beschickung eines dreidimensionalen Druckers vom Strang- oder Tintenstrahl-Typ verwendet werden,
umfassend einen Schritt des Formens des besagten Strangs mittels eines dreidimensionalen Druckers, um eine Vorrichtung, die in den menschlichen Körper implantiert werden kann, oder eine Abstandsvorrichtung zur Behandlung einer Infektion in einem Knochen oder einer Gelenkstelle zu erhalten,
worin die besagte Vorrichtung porös ist und, sobald sie geformt ist, in der Lage ist, eine andere pharmazeutische oder medizinische Substanz zu absorbieren, wobei letztere Gentamicinsulfat ist oder sich von dem in der besagten Vorrichtung enthaltenen Gentamicinsulfat unterscheidet.

10. Verfahren nach Anspruch 9, worin der besagte Misch- oder Zugabeschritt und der besagte Erhitzungsschritt des besagten Materials gleichzeitig in einer speziellen Maschine, das heißt einem Extruder, erfolgen.

## Revendications

1. Matériau biocompatible et implantable dans le corps humain sous la forme d'un fil utilisé pour alimenter une imprimante tridimensionnelle de type à fil ou jet d'encre, pour l'obtention d'un dispositif pouvant être implanté dans le corps humain ou d'un dispositif d'espacement pour le traitement d'une infection d'un os ou d'une articulation, comprenant une matière plastique comprenant du polyéthylène basse densité ou du polyéthylène haute densité ou du polyéthylène à très haut poids moléculaire (UHMWPE) ou un mélange de ceux-ci, **caractérisé en ce qu'**il comprend un additif, dans lequel ledit additif est une substance pharmaceutique ou médicale comprenant du sulfate de gentamicine, et un agent radio-opacifiant, **en ce que** ledit matériau biocompatible est moulable et il est extrudé dans ledit fil **et en ce que** ledit matériau biocompatible comprend un additif supplémentaire comprenant un matériau céramique soluble et/ou résorbable, sous forme de poudre ou de granulés, c'est-à-dire de phosphate tricalcique ou de sulfate de calcium ou d'hydroxyapatite.

2. Matériau selon la revendication 1, dans lequel ledit fil a un diamètre compris entre 1 et 10 mm ou entre 1 et 5 mm.

3. Matériau selon la revendication 1, dans lequel ledit matériau biocompatible est moulable au moyen d'une imprimante tridimensionnelle.

4. Matériau selon la revendication 1, dans lequel ledit agent radio-opacifiant est constitué de poudres métalliques de tungstène, de tantale ou d'argent ou de leurs sels ou de sulfate de baryum, d'oxyde de zirconium, d'oxyde de bismuth.

5. Matériau selon la revendication 1, dans lequel ladite matière plastique comprend en outre une résine acrylique comprenant un copolymère acrylique comprenant du MMA, du styrène et de l'acrylate d'éthyle ou du polyméthacrylate de méthyle ou des mélanges comprenant des polymères et/ou des copolymères acryliques.

6. Dispositif implantable dans le corps humain ou dispositif d'espacement pour le traitement d'une infection dans un os ou une articulation, comprenant un matériau biocompatible, dans lequel ledit matériau biocompatible comprend une matière plastique comprenant du polyéthylène basse densité ou du polyéthylène haute densité ou du polyéthylène à très haut poids moléculaire (UHMWPE) ou un mélange de ceux-ci, **caractérisé en ce que** ledit matériau biocompatible comprend un additif, dans lequel ledit additif est une substance pharmaceutique ou médicale comprenant du sulfate de gentamicine et un agent radio-opacifiant, dans lequel ledit dispositif est obtenu par extrusion dudit matériau biocompatible dans un fil, puis par moulage dudit fil au moyen d'une imprimante tridimensionnelle, **en ce que** ledit dispositif comprend un additif supplémentaire comprenant un matériau céramique soluble et/ou résorbable, sous forme de poudre ou de granulés, c'est-à-dire de phosphate tricalcique ou de sulfate de calcium ou d'hydroxyapatite, **et en ce que** ledit dispositif est poreux et, une fois formé, est capable d'absorber une autre substance pharmaceutique ou médicale, cette dernière étant du sulfate de gentamicine ou différente du sulfate de gentamicine contenu dans ledit dispositif.

7. Dispositif selon la revendication 6, dans lequel ledit agent radio-opacifiant est constitué de poudres ou de sels métalliques de tungstène, de tantale ou d'argent, de sulfate de baryum, d'oxyde de zirconium, d'oxyde de bismuth.

8. Dispositif selon l'une quelconque des revendications 6 ou 7, dans lequel ladite matière plastique comprend en outre une résine acrylique comprenant un copolymère composé de MMA, de styrène et d'acrylate d'éthyle ou de polyméthacrylate de méthyle ou des mélanges comprenant des polymères et/ou des copolymères acryliques.

9. Procédé d'obtention d'un dispositif pouvant être implanté dans le corps humain selon l'une quelconque des revendications 6 à 8 précédentes, comprenant les étapes suivantes :
fourniture d'une matière plastique sous forme de granulés comprenant du polyéthylène basse densité ou du polyéthylène haute densité ou du polyéthylène à très haut poids moléculaire (UHMWPE) ou un mélange de ceux-ci, dans lequel ladite matière peut être moulée,
fourniture d'un additif, dans lequel ledit additif est une substance pharmaceutique ou médicale comprenant du sulfate de gentamicine et un agent radio-opacifiant,
fourniture d'un additif supplémentaire comprenant une matière céramique soluble et/ou résorbable, sous forme de poudre ou de granulés, c'est-à-dire de phosphate tricalcique ou sulfate de calcium ou de l'hydroxyapatite,
mélange ou ajout de ladite matière plastique et dudit additif pour obtenir un matériau de base mélangé ou ajouté,
chauffage dudit matériau de base mélangé ou ajouté à une température prédéterminée et extrudage du matériau fondu sous la forme d'un ou plusieurs fils utilisés pour alimenter une imprimante tridimensionnelle de type à fil ou jet d'encre,
comprenant une étape de moulage dudit fil au moyen d'une imprimante tridimensionnelle, afin d'obtenir un dispositif pouvant être implanté dans le corps humain ou un dispositif d'espacement afin de traiter une infection d'un os ou d'une articulation,
dans lequel ledit dispositif est poreux et, une fois formé, est capable d'absorber une autre substance pharmaceutique ou médicale, cette dernière étant du sulfate de gentamicine ou différente du sulfate de gentamicine contenu dans ledit dispositif.

10. Procédé selon la revendication 9, dans lequel ladite étape de mélange ou d'ajout et ladite étape de chauffage dudit matériau se produisent simultanément dans une machine dédiée, c'est-à-dire une extrudeuse.
